(19) Europäisches Patentamt
European Patent Office
Office européen des brevets

(11) Publication number: **0 037 698**
**A1**

(12) **EUROPEAN PATENT APPLICATION**

(21) Application number: 81301392.7

(22) Date of filing: 31.03.81

(51) Int. Cl.[3]: C 07 C 47/55
C 07 C 45/49, C 07 C 33/46
C 07 C 25/18, C 07 C 59/66
C 07 C 69/66, C 07 C 51/347
C 07 C 67/31, C 07 C 29/14

(30) Priority: 03.04.80 GB 8011377

(43) Date of publication of application:
14.10.81 Bulletin 81/41

(84) Designated Contracting States:
AT BE CH DE FR GB IT LI LU NL SE

(71) Applicant: IMPERIAL CHEMICAL INDUSTRIES LIMITED
Imperial Chemical House Millbank
London SW1P 3JF(GB)

(72) Inventor: Bowie, Raymond Alexander
51 Park Mount Drive
Macclesfield Cheshire(GB)

(74) Representative: Smith, Stephen Collyer et al,
Imperial Chemical Industries Limited Legal Department: Patents Thames House North Millbank
London SW1P 4QG(GB)

(54) **Benzaldehyde derivatives for use as a chemical intermediate.**

(57) The invention provides the novel chemical intermediate 4-(4-chlorophenyl)benzaldehyde, or the hydrate thereof; processes for its production and for its conversion to the known compounds 4-(4-chlorophenyl)-benzyl alcohol and 4-(4-chlorophenyl)benzyl chloride and bromide.

These latter compounds are intermediates known for use in the manufacture of anti-arthritic benzyloxyacetic acids of the formula:

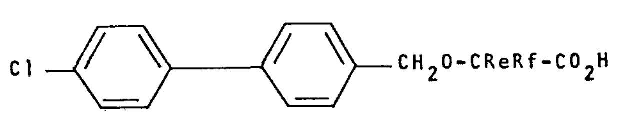

IV

in which Re is hydrogen or (1-4C)alkyl and Rf is (1-4C)alkyl or phenyl optionally bearing a halogeno substituent, and have previously only been obtainable by the use of potentially toxic chloromethyl ethers. The invention therefore also provides processes for the manufacture of the anti-arthritic acids of formula IV starting from 4-(4-chlorophenyl)benzaldehyde which avoid the use of such hazardous ethers.

EP 0 037 698 A1

Croydon Printing Company Ltd.

Benzaldehyde Derivatives for Use as a Chemical Intermdediate

This invention relates to a novel benzaldehyde derivative which is useful as a chemical intermediate, to a process for its manufacture, and to processes for its conversion to valuable end-products. More particularly the invention concerns the novel compound 4-(4-chlorophenyl)benzaldehyde, its production from 4-chlorobiphenyl and its conversion to 4-(4-chlorophenyl)benzyl alcohol and 4-(4-chlorophenyl)-benzyl chloride which are themselves useful intermediates in the manufacture of known anti-arthritic benzyloxyacetic acid derivatives.

The production of various p-substituted benzaldehyde derivatives is described in West German Offenlegungsschrift No. 2801887. However there is no mention of the production of the compound of formula I below.

According to the invention there is provided the novel chemical intermediate of the formula:-

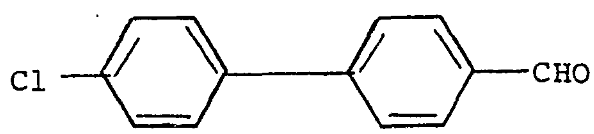

I

known as 4-(4-chlorophenyl)benzaldehyde, or the hydrate thereof.

The invention further provides a process for the manufacture of the intermediate of formula I which comprises reacting 4-chlorobiphenyl with a mixture of carbon monoxide and hydrogen chloride in the presence of aluminium chloride; optionally followed by conventional hydrate formation.

It will be appreciated that this process is an example of the well known Gatterman-Koch reactions (reviewed by Olah and Kulin in "Friedel-Crafts and

related reactions" Vol. III, p.1154-1162, Interscience New York, 1963) and may be carried out under the standard conditions well known in the art for that reaction. Accordingly, the process is generally carried out under a pressure in the range, for example, 70 to 250 bars and at a temperature in the range, for example, 20 to 50°C. Alternatively, the process may be carried out in the presence of cuprous chloride as a promoter or carrier in which case a reduced pressure in the range, for example, 1 to 5 bars and a temperature in the range, for example, 20 to 100°C. may be employed. In either case the process may conveniently be carried out in the presence of a suitable solvent or diluent, for example carbon disulphide, benzene, chlorobenzene or nitrobenzene.

The hydrogen chloride and carbon monoxide may be provided from separate pressurised cylinders. Alternatively, they may be conveniently provided as an equi-molar mixture by the reaction of chlorosulphonic acid with formic acid, or by the reaction of benzoyl chloride with formic acid. As a further alternative, the necessary hydrogen chloride may be produced in situ by the reaction of aluminium chloride with water (since less than stoichiometric quantities of hydrogen chloride may be used in the process) and the carbon monoxide provided from an external pressurised cylinder.

The invention also provides a further process for the manufacture of the intermediate of formula I or a hydrate thereof which comprises reacting 4-chlorobiphenyl with hexamethylenetetramine in the process of a strong acid; optionally followed by hydrate formation.

A particularly suitable strong acid is, for example, trifluoroacetic acid, which may also conveniently function as solvent for the process. The process is conveniently performed at a temperature in the range, for example, 20-100°C.

The intermediate of formula I may be converted to the known chemical intermediate 4-(4-chlorophenyl)-benzyl alcohol by a conventional reduction process, which process is provided as a further feature of the invention.

Suitable reducing conditions are provided by, for example, alkali metal complex hydrides such as sodium or potassium borohydride or lithium aluminium hydride, catalytic hydrogenation such as hydrogenation in the presence of palladium-charcoal, sodium dithionite, and reducing metal systems, such as those based on iron, nickel or zinc.

The reduction may be carried out under the standard conditions well known for the above mentioned reagents. Thus, in general, the reduction is carried out at a temperature in the range, for example, 20 to 100°C., and in the presence of a suitable solvent or diluent, for example water, ethanol, 2-propanol or acetic acid, or a mixture of two or more thereof; it being understood that when lithium aluminium hydride is used as reducing agent, a non-aqueous solvent or diluent, such as tetrahydrofuran must be used.

The starting intermediate of formula I may conveniently be reduced in the above process immediately following its formation in the Gatterman-Koch reaction and without being separately purified. In which case, no additional solvent need be added, but it is desirable to add a catalytic amount of a suitable quaternary ammonium salt such as cetyltrimethylammonium chloride as a phase transfer catalyst.

4-(4-Chlorophenyl)benzyl alcohol and the corresponding benzyl chloride are intermediates known for use in the synthesis of anti-arthritic benzyloxyacetic acid derivatives:

(a) of the general formula:-

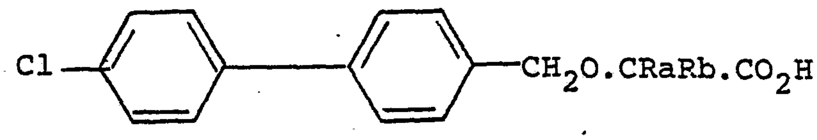 II

wherein Ra and Rb are independently hydrogen or (1-4C)alkyl radicals, and the base-addition salts and simple esters thereof, and in particular of the compound wherein Ra and Rb are both methyl radicals (UK patent specification Serial No 1,140,748); and (b), of the general formula:-

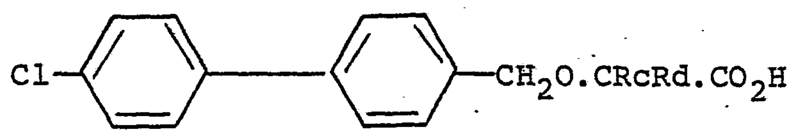 III

wherein Rc is hydrogen or a (1-4C)alkyl radical, and Rd is a phenyl radical optionally bearing a halogeno radical, and the base-addition salts and simple esters thereof, and in particular of the compounds wherein Rc is a methyl or ethyl radical and Rd is a phenyl radical (European patent application,publication No. 9306Al).

4-(4-Chlorophenyl)benzyl alcohol has hitherto only been obtained by hydrolysis of 4-(4-chlorophenyl)-benzyl chloride which was itself obtained by reaction of 4-chlorobiphenyl with chloromethyl ethers (UK patent specification Serial No. 1,140,748). Although this latter procedure is satisfactory for small-scale manufacture, it is unsuited to large-scale manufacture (except in expensive, specially built plant) because of the difficulties associated with handling the potentially toxic chloromethyl ethers.

Further, since 4-(4-chlorophenyl)benzyl alcohol may be readily converted by reaction with conventional chlorinating or brominating agents well known in the art

(for example thionyl chloride, phosphorus tribromide or phosphoryl bromide) into the corresponding benzyl chloride or bromide, the use of the intermediate of formula I also allows these latter compounds to be obtained if necessary on a large-scale from 4-chlorobiphenyl without the use of hazardous chloromethyl ethers.

The latter process is also provided as a feature of the invention.

According to a further feature of the invention there is provided a process for the manufacture of a compound of the formula:-

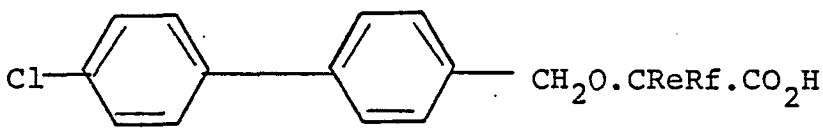

IV

wherein Re is hydrogen or a (1-4C)alkyl radical, and Rf is a (1-4C)alkyl radical or a phenyl radical optionally bearing a halogen radical, or a base-addition salt or a (1-4C)alkyl ester thereof; which comprises carrying out the known steps of reacting a base-addition salt of 4-(4-chlorophenyl)benzyl alcohol with a halogenated compound of the formula:-

$$Hal.CReRf.CO_2Rg \qquad V$$

wherein Re and Rf have the meanings defined above, Rg is hydrogen or a (1-4C)alkyl radical, and Hal. is a chloro, bromo or iodo radical, followed if necessary, by hydrolysis to the carboxylic acid, base-addition salt formation or (1-4C)alkyl ester formation depending on the nature of Rg; said process characterised in that

the 4-(4-chlorophenyl)benzyl alcohol is obtained by reduction of the intermediate of formula I, for example using a procedure specified hereinbefore.

The known steps in the above process are described in UK patent specificaiton Serial No. 1,140,748, and European patent application, publication No. 9306A1, which documents are incorporated herein by reference.

However, the necessary base-addition salts of 4-(4-chlorophenyl)benzyl alcohol are, for example, alkali metal salts such as the sodium or potassium salt and a preferred halogenated compound of formula V is, for example, ethyl 2-bromo-2-methylpropionate when the compound 2-[4-(4-chlorophenyl)benzyloxy]-2-methylpropionic acid, or a base-addition salt thereof, is required as final product.

The reaction with the halogenated compound of formula V may be carried out at a temperature in the range, for example, 20-100°C. and in a solvent or diluent, for example, chlorobenzene, toluene, xylene or dimethylformamide, or in a mixture of two or more thereof.

Rg is preferably a methyl, ethyl or butyl radical, and the necessary hydrolysis step in such a case is preferably carried out using an aqueous alkali metal hydroxide, for example aqueous sodium or potassium hydroxide, at a temperature in the range, for example 20 to 100°C., and optionally in the presence of a solvent or diluent, for example ethanol, methanol or 2-propanol. The initially formed alkali metal salt may be converted to the free acid by a conventional acidification procedure.

The process is of particular value for the production of that compound of formula IV in which Re and Rf are both methyl radicals, or non-toxic base-addition salts thereof.

According to a further feature of the invention there is provided a process for the manufacture of a compound of the formula IV wherein Re and Rf have the meanings defined above, or a base-addition salt or a (1-4C)alkyl ester thereof; which comprises carrying out the known steps of reacting 4-(4-chlorophenyl)benzyl chloride or bromide with a base-addition salt of a compound of the formula:-

$$HO.CReRf.CO_2Rg \qquad VI$$

wherein Re, Rf and Rg have the meanings defined above, followed if necessary, by hydrolysis to the carboxylic acid, base-addition salt formation or (1-4C)alkyl ester formation, depending on the nature of Rg; said process characterised in that the 4-(4-chlorophenyl)benzyl chloride or bromide is obtained by reduction of the intermediate of formula I to give 4-(4-chlorophenyl)-benzyl alcohol which is then reacted with a suitable chlorinating or brominating agent, for example using a procedure specified hereinbefore.

The known steps in the above process are also described in UK patent specification Serial No. 1,140,748 and European patent application, publication No. 9306A1. However, suitable base-addition salts of the compounds of formula VI are, for example, alkali metal salts such as the sodium or potassium salts, and when Rg is hydrogen, di-salts such as di-alkali metal salts are preferred. The salts may conveniently be formed by reaction of the compound of formula VI with an appropriate base, such as sodium or potassium hydroxide or hydride, prior to the main process. The reaction of the 4-(4-chlorophenyl)benzyl chloride or bromide with a base-addition salt of a compound of formula VI may be generally carried out in a solvent or

diluent, such as chlorobenzene, toluene, xylene or dimethylformamide, or in a mixture of two or more thereof, and at a temperature in the range, for example, 20-150°C., and more particularly, 60-120°C. Preferred compounds of formula VI are, for example, the ethyl esters of 2-hydroxy-2-phenylpropionic acid, 2-hydroxy-2-phenylbutyric acid and 2-hydroxy-2-methylpropionic acid.

Preferred values for Rg and suitable hydrolysis conditions are, for example, as described for the preceding process.

The process is of particular value for the production of those compounds of formula IV wherein Re and Rf are both methyl radicals; or Re is a methyl or ethyl radical and Rf is a phenyl radical, or a base-addition salt thereof.

The invention is illustrated by the following non-limiting Examples in which:-

(i) all evaporations unless otherwise stated were carried out under reduced pressure; and

(ii) yields, where given, are purely illustrative and are not to be construed as the maximum attainable.

Example 1

A mixture of 4-chlorobiphenyl (18.8 g.) and aluminium chloride (29.5 g.) was stirred in chlorobenzene (150 ml.) at 55-60°C., in a pressure vessel. The vessel was pressurised to 30 psi (2.1 bar) with an equimolar mixture of carbon monoxide and hydrogen chloride (prepared by the action of 98-100%formic acid on chlorosulphonic acid). These reaction conditions were maintained for a period of 4 days. After this time the reaction mixture was added, with stirring, to a mixture of ice (500 g.) and water (500 g.). The organic phase was

extracted with chlorobenzene (50 ml.). The combined organic phases were then washed successively with 2M hydrochloric acid (100 ml.) and water (2 x 100 ml.).

The organic phase [containing 4-(4-chlorophenyl)benzaldehyde] was then stirred with an aqueous solution of sodium borohydride (1.2 g. 0.03 moles) in water (20 ml.) containing cetyltrimethylammonium bromide (0.1 g.) and potassium hydroxide (0.1 g.) for 3 hours at 35-40°C. After the reduction was complete the excess sodium borohydride was destroyed by the careful addition of a small amount of dilute hydrochloric acid. The organic phase was then washed with water (2 x 100 ml.) and the organic solvent removed by azeotropic distillation with water. The aqueous slurry of crude product was filtered and the precipitate was washed with water (100 ml.) and dried. The solid (17.2 g.) obtained was recrystallised from toluene/cyclohexane to give 4-(4-chlorophenyl)benzyl alcohol (m.p. 134-135°C.) in 52% overall yield.

Example 2

To a solution of 4-chlorobiphenyl (18.8 g.) in chlorobenzene (75 ml.) contained in a pressure vessel was added aluminium chloride (26.6 g.), cuprous chloride (3.7 g.) and water (0.25 ml.). The vessel was evacuated to approximately 0.0267 bar, agitated and pressurised to approximately 2.1 bar with carbon monoxide. The reaction mixture was then heated at approximately 40°C. for 30 hours, additional carbon monoxide being added as required to keep the pressure constant.

The reaction mixture was then poured, with stirring, into cold water (250 ml.) and the organic phase separated and washed consecutively with equal volumes of 10% w/v sodium acetate solution and then water. The organic phase was then separated, dried ($Na_2SO_4$) and evaporated onto chromatographic silica gel (40 g.). The residue obtained was added to the top of a column of

chromatographic silica gel (160 g.) made up in carbon tetrachloride. The column was first eluted with carbon tetrachloride and then with chloroform. The initial fractions (2 x 50 ml.) (containing mainly residual 4-chlorophenyl and chlorobenzene) were discarded. Subsequent fractions were combined and evaporated to give 4-(4-chlorophenyl)benzaldehyde, as a solid in 43% yield [m.p. 117-119$^{o}$C. after recrystallisation from acetonitrile; NMR ($CDCl_3$; 100 $MH_z$) $\delta$ 7.35-7.65 (multiplet, 4 aromatic protons); 7.87 (AB quartet, 4 aromatic protons, J=9$H_z$); 10.05 (singlet, 1 aldehydic proton) ppm (relative to TMS)].

Example 3

Thionyl chloride (41.8 g.) was added dropwise during 2 hours to a stirred suspension of 4-(4-chlorophenyl)benzyl alcohol (50 g.) in toluene (500 ml.) and N,N-dimethylformamide (5 ml.) maintained at 18-22$^{o}$C. The resulting solution was stirred for a further hour and then added to a stirred solution of sodium acetate (200 g.) in water (1 l.). The organic phase was washed with water (500 ml.), dried ($Na_2SO_4$) and evaporated to give 4-(4-chlorophenyl)benzyl chloride as a solid in 50% yield [m.p. 66-69$^{o}$C., after recrystallisation from petroleum ether (b.p. 60-80$^{o}$C.)].

Example 4

A solution of methyl 2-hydroxy-2-phenylbutyrate (8.9 g.) in dry dimethylformamide (9 ml.) was added to a stirred suspension of sodium hydride (2.13 g.; 57% dispersion in mineral oil) in dry dimethylformamide (40 ml.) over 45 minutes, under an atmosphere of nitrogen. The temperature of the solution rose to 44$^{o}$C, and after stirring for a further 2 hours, a solution of 4-(4-chlorophenyl)benzyl chloride (10.9 g.) in cyclohexane (108 ml.) was added and the solution was stirred at room temperature for 70 hours. Formic acid (1.8 ml.) was added and the

solution was poured into water (60 ml.). The organic phase was separated and washed with water (60 ml.). Methanol (7 ml.) and sodium hydroxide (3.7 g.) were added to the separated organic phase and the mixture was heated under reflux for 2 hours. Cyclohexane (12.5 ml.) was added and the stirred mixture was heated to distillation. After collection of 16.5 ml. of the distillate the mixture was cooled to 20 to 25°C The solid which formed was collected, washed with cyclohexane (75 ml.), dried and added to a stirred mixture of ethyl acetate (100 ml.) and 2M hydrochloric acid (100 ml.). The organic phase was separated, washed with water (50 ml). and evaporated to give an oil which after recrystallisation from a mixture of ethyl acetate (11 ml.) and petroleum spirit (b.p. 60-80°C.; 79ml.) gave 2-[4-(4-chlorophenyl)benzyloxy]-2-phenylbutyric acid (12.7 g.), m.p. 128-130°C.

Example 5

Sodium hydride (1.0 g. of 60% w/w dispersion in oil) was added to dry N,N-dimethylformamide (65 ml.) under a nitrogen atmosphere. A solution of the methyl 2-hydroxyisobutyrate (2.55 g.) in N,N-dimethylformamide (15 ml.) was added dropwise to the stirred suspension during 30 minutes maintaining the temperature in the range 20 to 25°C. The resultant solution was stirred for a further 1 hour. A solution of 4-(4-chlorophenyl)-benzyl chloride (5.15 g.) in dry N,N-dimethylformamide (20 ml.) was added to the reaction mixture during 30 minutes maintaining the temperature in the range 20 to 25°C. The subsequent mixture was stirred for 18 hours at 30°C under a nitrogen atmosphere. The precipitate was removed by filtration and the filtrate evaporated. The resultant oil was mixed with ethanol (100 ml.), water (20 ml.) and caustic liquor (4 ml.) and then heated at 100°C. for 1 hour. The mixture

was again evaporated and the residue was partitioned three times between water (500 ml.) and toluene (100 ml.) The aqueous phase was steam distilled to remove any remaining toluene and acidified with concentrated hydrochloric acid ( ca 5 ml.) to give 2-[4-(4-chlorophenyl)benzyloxy]-2-methylpropionic acid as a white solid (3.5 g.)., m.p. 141-145°C.

Example 6

Trifluoroacetic acid (150 ml.) was added slowly to a mixture of 4-chlorobiphenyl (18.9 g.) and hexamethylenetetramine (28.0 g.). An exothermic reaction occurred with the temperature of the mixture rising to 50 to 70°C. The mixture was heated under reflux for 12 to 16 hours, the progress of the reaction being followed by thin layer chromatography ($SiO_2$: $CHCl_3$). The solution obtained was concentrated to low volume *in vacuo* and the residual liquors added to an excess of ice-water. The mixture was basified to pH 9-10 with solid sodium carbonate and then extracted with ether (3 x 200 ml.). The extracts were dried ($MgSO_4$) and evaporated. The solid residue obtained was recrystallised once from petroleum ether (b.p. 60-80°C) and once from acetonitrile to give 4-(4-chlorophenyl)benzaldehyde as a solid in 30% yield, having an identical NMR spectrum to that described in Example 2.

Claims

What is claimed is:-

1. 4-(4-Chlorophenyl)benzaldehyde having the formula:-

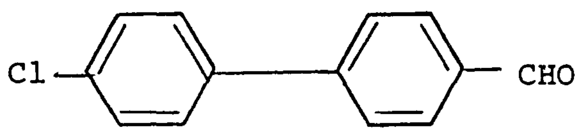

I

or the hydrate thereof.

2. A process for the manufacture of 4-(4-chlorophenyl)benzaldehyde, or the hydrate thereof, characterised by formylating 4-chlorobiphenyl.

3. A process according to claim 2 characterised in that the formylation is performed by reacting 4-chlorobiphenyl (a), with a mixture of carbon monoxide and hydrogen chloride in the presence of aluminium chloride; or (b), with hexamethylenetetramine in the presence of a strong acid; optionally followed by hydrate formation.

4. A process according to part (a) of claim 3 characterised in that the reaction is performed in the presence of cuprous chloride as a carrier or promoter.

5. A process according to part (b) of claim 3 characterised in that the strong acid is trifluoroacetic acid.

6. A process for the manufacture of 4-(4-chlorophenyl)benzylalcohol which comprises reducing 4-(4-chlorophenyl)benzaldehyde or the hydrate thereof by a known procedure.

7. A process for the manufacture of 4-(4-chlorophenyl)benzyl chloride or bromide which comprises carrying out in sequence the steps of (i), reducing 4-(4-chlorophenyl)benzaldehyde or the hydrate thereof,

and (ii), reacting the thus formed 4-(4-chlorophenyl)-benzyl alcohol with a conventional chlorinating or brominating agent; in both steps using known procedures.

8. A process for the manufacture of a compound of the formula:-

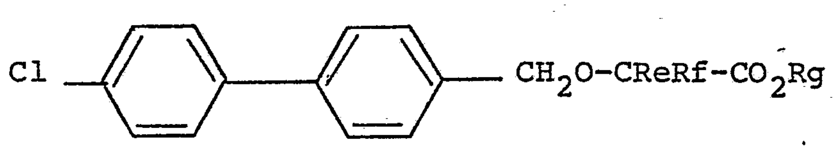 IV

wherein Re is hydrogen or a (1-4C)alkyl radical, and $R_f$ is a (1-4C)alkyl radical or a phenyl radical optionally bearing a halogeno radical, or a base-addition salt or a (1-4C)alkyl ester thereof; which comprises carrying out the known steps of reacting a base-addition salt of 4-(4-chlorophenyl)benzyl alcohol with a halogenated compound of the formula:-

$$\text{Hal-CReRf-CO}_2\text{Rg} \qquad \text{V}$$

wherein Re and Rf have the meanings defined above, Rg is hydrogen or a (1-4C)alkyl radical, and Hal. is a chloro, bromo or iodo radical, followed if necessary by hydrolysis to the carboxylic acid, base-addition salt formation or (1-4C)alkyl ester formation depending on the nature of Rg; said process characterised in that the 4-(4-chlorophenyl)benzyl alcohol is obtained by reduction of 4-(4-chlorophenyl)benzaldehyde or the hydrate thereof by a known procedure.

9. A process according to claim 8 characterised in that in the starting material of formula V Re and Rf are both methyl radicals.

10. A process for the manufacture of a compound of the formula:-

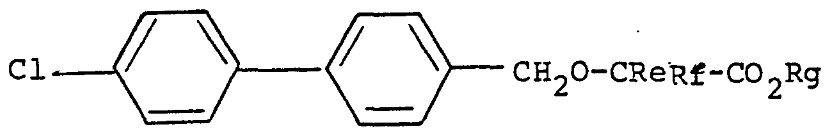

IV

wherein Re is hydrogen or a (1-4C)alkyl radical, and Rf is a (1-4C)alkyl radical or a phenyl radical optionally bearing a halogen radical, or a base-addition salt or (1-4C)alkyl ester thereof which comprises carrying out the known steps of reacting 4-(4-chlorophenyl)benzyl chloride or bromide with a base addition salt of a compound of the formula:-

$$HO-CReRf-CO_2Rg \qquad VI$$

wherein Re, Rf and Rg have the meanings defined above, followed if necessary, by hydrolysis to the carboxylic acid, base-addition salt formation or (1-4C)alkyl ester formation, depending on the nature of Rg; said process characterised in that the 4-(4-chlorophenyl)-benzyl chloride or bromide is obtained by reduction of 4-(4-chlorophenyl)benzaldehyde or the hydrate thereof to give 4-(4-chlorophenyl)benzyl alcohol which is then reacted with a suitable chlorinating or brominating agent; in both cases, using known procedures.

11. A process according to claim 10 characterised in that in the starting material of formula VI Re and Rf are both methyl radicals, or Re is a methyl or ethyl radical and Rf is a phenyl radical.

SCS/ML: 20 Feb 81
PH 31249

Authorised Representative

0037698

PH 31249/EP

Claims for Austria

What is claimed is:-

1. A process for the manufacture of 4-(4-chlorophenyl)benzaldehyde having the formula:-

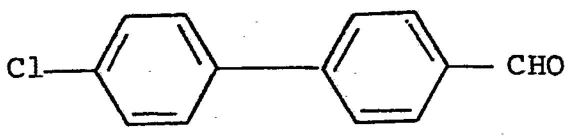

I

or the hydrate thereof characterised by formylating 4-chlorobiphenyl.

2. A process according to claim 1 characterised in that the formylation is performed by reacting 4- chlorobiphenyl (a), with a mixture of carbon monoxide and hydrogen chloride in the presence of aluminium chloride; or (b), with hexamethylenetetramine in the presence of a strong acid; optionally followed by hydrate formation.

3. A process according to part (a) of claim 2 characterised in that the reaction is performed in the presence of cuprous chloride as a carrier or promoter.

4. A process according to part (b) of claim 2 characterised in that the strong acid is trifluoroacetic acid.

5. A process for the manufacture of 4-(4-chlorophenyl)benzylalcohol which comprises reducing 4-(4-chlorophenyl)benzaldehyde or the hydrate thereof by a known procedure.

6. A process for the manufacture of 4-(4-chlorophenyl)benzyl chloride or bromide which comprises carrying out in sequence the steps of (i), reducing 4-(4-chlorophenyl)benzaldehyde or the hydrate thereof,

and (ii), reacting the thus formed 4-(4-chlorophenyl)-benzyl alcohol with a conventional chlorinating or brominating agent; in both steps using known procedures.

7. A process for the manufacture of a compound of the formula:-

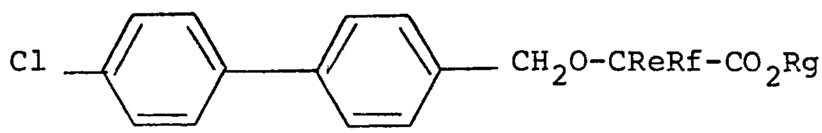

IV

wherein Re is hydrogen or a (1-4C)alkyl radical, and $R_f$ is a (1-4C)alkyl radical or a phenyl radical optionally bearing a halogeno radical, or a base-addition salt or a (1-4C)alkyl ester thereof; which comprises carrying out the known steps of reacting a base-addition salt of 4-(4-chlorophenyl)benzyl alcohol with a halogenated compound of the formula:-

$$Hal\text{-}CReRf\text{-}CO_2Rg \qquad V$$

wherein Re and Rf have the meanings defined above, Rg is hydrogen or a (1-4C)alkyl radical, and Hal. is a chloro, bromo or iodo radical, followed if necessary by hydrolysis to the carboxylic acid, base-addition salt formation or (1-4C)alkyl ester formation depending on the nature of Rg; said process characterised in that the 4-(4-chlorophenyl)benzyl alcohol is obtained by reduction of 4-(4-chlorophenyl)benzaldehyde or the hydrate thereof by a known procedure.

8. A process according to claim 7 characterised in that in the starting material of formula V Re and Rf are both methyl radicals.

9. A process for the manufacture of a compound of the formula:-

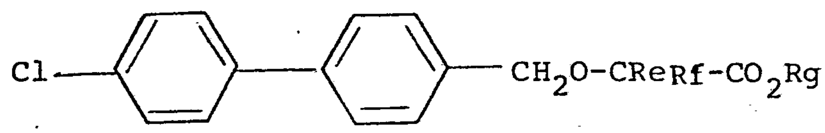

IV

wherein Re is hydrogen or a (1-4C)alkyl radical, and Rf is a (1-4C)alkyl radical or a phenyl radical optionally bearing a halogen radical, or a base-addition salt or (1-4C)alkyl ester thereof which comprises carrying out the known steps of reacting 4-(4-chlorophenyl)benzyl chloride or bromide with a base addition salt of a compound of the formula:-

HO-CReRf-$CO_2$Rg VI

wherein Re, Rf and Rg have the meanings defined above, followed if necessary, by hydrolysis to the carboxylic acid, base-addition salt formation or (1-4C)alkyl ester formation, depending on the nature of Rg; said process characterised in that the 4-(4-chlorophenyl)-benzyl chloride or bromide is obtained by reduction of 4-(4-chlorophenyl)benzaldehyde or the hydrate thereof to give 4-(4-chlorophenyl)benzyl alcohol which is then reacted with a suitable chlorinating or brominating agent; in both cases, using known procedures.

10. A process according to claim 9 characterised in that in the starting material of formula VI Re and Rf are both methyl radicals, or Re is a methyl or ethyl radical and Rf is a phenyl radical.

SCS/ML: 20 Feb 81

PH 31249/OEE

European Patent Office

**0037698**

Application number

# EUROPEAN SEARCH REPORT

EP 81301392.7

| DOCUMENTS CONSIDERED TO BE RELEVANT | | | CLASSIFICATION OF THE APPLICATION (Int. Cl.³) |
|---|---|---|---|
| Category | Citation of document with indication, where appropriate, of relevant passages | Relevant to claim | |
| | US - A - 3 979 468 (COLIN FREDRICK WEBB)<br>+ Column 3, lines 1-5 +<br>-- | 1 | C 07 C 47/55<br>C 07 C 45/49<br>C 07 C 33/46<br>C 07 C 25/18<br>C 07 C 59/66<br>C 07 C 69/66<br>C 07 C 51/347<br>C 07 C 67/31<br>C 07 C 29/14 |
| | GB - A - 985 650 (IMPERIAL SMELTING CORPORATION)<br>+ Example 1 +<br>-- | 6 | TECHNICAL FIELDS SEARCHED (Int. Cl.³) |
| D | GB - A - 1 140 748 (IMPERIAL CHEMICAL INDUSTRIES LIMITED OF IMPERIAL CHEMICAL HOUSE)<br>+ Examples 1,2 +<br>---- | 8-11 | C 07 C 29/00<br>C 07 C 47/00<br>C 07 C 45/00<br>C 07 C 31/00<br>C 07 C 33/00<br>C 07 C 25/00<br>C 07 C 59/00<br>C 07 C 69/00<br>C 07 C 51/00<br>C 07 C 67/00 |

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| VIENNA | 30-06-1981 | HEIN |